# EUROPÄISCHE PATENTSCHRIFT

(11) **EP 0 525 388 B1**
(45) Veröffentlichungstag und Bekanntmachung des Hinweises auf die Patenterteilung: **20.09.1995**
(21) Anmeldenummer: 92110680.3
(22) Anmeldetag: 25.06.1992
(51) Int. Cl.: A61K 9/00, A61K 9/46

(54) **Lutsch- oder Kautablette**
Suckable or chewable tablet
Comprimé à sucer ou à mâcher

(30) Priorität: 01.07.1991 CH 1943/91
(43) Veröffentlichungstag der Anmeldung: 03.02.1993
(73) Patentinhaber: Gergely, Gerhard, Dr., A-1053 Wien (AT)
(72) Erfinder: Gergely, Gerhard, Dr., A-1053 Wien (AT); Gergely, Thomas, Dr., A-1053 Wien (AT); Gergely, Irmgard, A-1053 Wien (AT)
(74) Vertreter: Büchel, Kurt F., Dr.

(56) Entgegenhaltungen:
- EP-A- 0 233 853
- WO-A-91/07174
- FR-A- 1 484 202
- US-A- 2 984 543
- US-A- 3 887 700

## Beschreibung

Bekannte Lutsch- oder Kautabletten, insbesondere solche mit pharmazeutischen Wirkstoffen, haben im allgemeinen einen faden Geschmack; insbesondere Tabletten, die als Wirkstoff Calciumcarbonat oder Magnesiumsalze enthalten, haben einen kreidigen oder unangenehmen Nachgeschmack. Daher hat man auch schon Tabletten oder direkt einzunehmende Granulate vorgeschlagen, die eine leichte Brausewirkung aufweisen, indem geringe Mengen einer Brausemischung, z.B. aus einer essbaren, organischen Säure und einem Alkali- und/oder Erdalkalicarbonat und/oder -bicarbonat zugesetzt werden. Wird eine grössere Menge an Brausebestandteilen, wie Säure und Bicarbonate, zugesetzt, so wird von vielen Menschen der starke Brauseeffekt als unangenehm bzw. auch als zu sauer empfunden, da die Säure beim Lutschen bzw. Kauen sich zuerst anlöst und erst anschliessend mit den Bicarbonaten oder Carbonaten reagiert. Ein weiterer Nachteil ist, dass es durch die Restfeuchtigkeit in der Tablette im Laufe der Lagerung zu einer unerwünschten Reaktion der Brausekomponenten kommt, wodurch die Lagerstabilität beeinträchtigt wird. Werden nur geringe Mengen an Brausekomponenten zugesetzt, so kann durch diese Reaktion im Laufe der Lagerung der Brauseeffekt fast völlig verschwinden.

Die Erfindung hat sich daher die Aufgabe gestellt, eine Lutsch- oder Kautablette, insbesondere für die Verabreichung pharmazeutischer Wirkstoffe, wie auch zur Behebung von Mangelerscheinungen, insbesondere in Form von Calcium- oder Magnesiumgaben, zu schaffen, die die obgenannten Nachteile vermeidet, beim Einnehmen ein durchwegs angenehmes Gefühl ergibt und auch einen angenehmen Nachgeschmack hinterlässt. Dies gelingt erfindungsgemäss durch die Verwirklichung der im Kennzeichen des Anspruches 1 beschriebenen Massnahmen. Weiter verbesserte Ausführungsformen der Erfindung sind in den Kennzeichen der abhängigen Ansprüche beschrieben.

Die Herstellung der erfindungsgemässen Tablette kann ähnlich wie in der DE-C2-3627475 beschrieben erfolgen, nur lässt man Zitronensäure und Calciumcarbonat nicht wie dort nur bis zu einem gewissen Zeitpunkt reagieren, sondern zu etwa zwei Drittel durchreagieren. Das verbleibende Drittel der sauren Gruppen reagiert nun beim Kauen oder Lutschen im Mund mit überschüssigem freiem Carbonat, insbesondere unter Einwirkung von Feuchtigkeit und der Körpertemperatur von etwa 37°C, unter ganz geringfügigem Prickeln; infolge der Ausbildung einer konzentrierten Lösung und durch die Gegenwart des bremsenden Hydrokolloids erfolgt aber keineswegs eine Durchreaktion, nicht einmal bis zum Schlucken der Bestandteile.

Grundsätzlich kann man auch Calciumcitrat bzw. Tri-Magnesiumdicitrat mit geringfügigen Mengen Carbonat und entweder geringen Säuremengen, oder einem sauren Calcium- oder Magnesiumcitrat, sowie den restlichen Tablettenbestandteilen mischen und erzielt damit auch einen ähnlichen, wenn auch nicht ganz so vorteilhaften Effekt wie nach der zuerst geschilderten Vorgehensweise, weil das Carbonat dann nicht, wie im ersten Fall, innerhalb der ausgebildeten Granulatkörner eingeschlossen ist und daher von innen heraus intensiv wirken kann. Trotzdem mag jedoch für bestimmte Anwendungsfälle die zweite Variante einer einfachen Mischung ausreichen. Die Erfindung bietet jedoch den Vorteil, die entsprechenden Alkali- oder Erdalkalisalze im Zuge der Granulierung im Verfahrensschritt ad hoc aus den billigen Carbonaten, wie z.B. Calcium- und/oder Magnesiumcarbonaten, herzustellen; man muss dann nicht die schwer erhältlichen und zudem teuren zweibasischen Salze einsetzen. Dabei kann man durch die Reaktion die Menge des für das jeweilige Produkt optimalen Prozentsatzes an zu bildenden Salzen steuern.

Besonders zweckmässig hat sich die erfindungsgemässe Tablette für die kombinierte Medikation von Calcium mit Fluor gezeigt. Es ist damit möglich, eine Kautablette mit z.B. 500 mg Calcium in Kombination mit z.B. einer Retardform von Natriummonofluorophosphat (entsprechend 75 bis 200 mg Natriummonofluorophosphat) herzustellen und somit eine angenehme und entsprechende Darreichungsform zu erzielen. Die jeweils gewünschte Dosierung kann in Form von Partikeln einer Korngrösse von 0,1 bis 0,5 mm in einer erfindungsgemässen Tablette geeigneter Grösse ohne Schwierigkeiten untergebracht werden, was z.B. in einer aus einer herkömmlichen Brausetablette bereiteten Trinklösung nur schwer zu bewerkstelligen ist, da die retardierten Teilchen des Wirkstoffes nur schwer zur Suspension zu bringen, bzw. in Schwebe zu halten sind.

Auch die für bestimmte Therapien besonders erwünschte Kombination von Acetylsalicylsäure mit Magnesium lässt sich in der erfindungsgemässen Darreichungsform besonders zweckmässig unterbringen.

### Beispiel 1:

Man legt in einen Vakuumkessel, dessen Manteltemperatur 90°C beträgt, 1250 Gewichtsteile feinpulvriges Calciumcarbonat vor und saugt anschliessend 125 Gewichtsteile Wasser ein, die 5 Minuten unter Mischen verteilt werden. Anschliessend werden 970 Gewichtsteile pulverisierte Zitronensäure und 330 Gewichtsteile Äpfelsäure zugefügt. Man evakuiert auf 700 mbar und lässt reagieren, bis das Vakuum einen Wert von 950 mbar aufweist. Diese definierte Reaktion erfolgt während 10 Minuten, bis die Masse die Temperatur von 60°C erreicht hat. In einem weiteren Schritt saugt man 60 Gewichtsteile Wasser ein und lässt 5 Minuten reagieren; sodann wird der Vorgang mit 50 ml während 15 Minuten wiederholt. Anschliessend fügt man 50 Gewichtsteile eines Guar-Gum zu und lässt die definierte Reaktion 15 Minuten unter Rühren weiterlaufen. Das Produkt wird abschliessend mittels Vakuum unter langsamem Rühren getrocknet und über ein Sieb ausgetragen. Aus dem erhaltenen Granulat werden unter Zusatz von Kohlehydraten, Zerfallsmitteln sowie Süss- und Aromastoffen, Tabletten von je 3,36 g verpresst, wobei z.B. folgende Mengen an Zusatzstoffen zum Einsatz kommen können (Mengen in mg):
2500 Granulat
200 Mannit
300 Fruchtzucker
100 Polyvinylpolypyrrolidon (Polyplasdone XL (R)
100 Reisstärke sowie Süss- und Aromastoffe

### Beispiel 2:

Zu dem wie in Beispiel 1 hergestellten Granulat kann auch ein retardiertes Natriummonofluorophosphat (entsprechend 50 bis 200 mg Natriummonofluorophosphat) zugefügt und mit den entsprechenden Zusatzstoffen, wie Süss-Stoffen, Füllstoffen und Aromen zu Tabletten verpresst werden.

### Beispiel 3:

In analoger Weise wie in Beispiel 1 werden 1388 Gewichtsteile basisches Magnesiumcarbonat in den Vakuummischer gefüllt, anschliessend 400 Gewichtsteile Wasser eingesaugt und während 10 Min. unter schwingendem Mischen verteilt. Eine Gesamtmenge von 1930 Gewichtsteilen pulverisierter Zitronensäure wird in vier gleichen Teilen zugefügt. Nach Einbringen jeden Teiles wird 15 bis 20 Min. unter schwingendem Mischen die Reaktion definiert zwischen 700 und 950 mbar gesteuert; die Temperatur der Füllung schwankt dabei zwischen 75 bis 86°C. Vor dem Zufügen der dritten und/oder vierten Charge wird zweckmässig einige Minuten lang auf 100 bis 300 mbar evakuiert und dadurch getrocknet. Anschliessend werden 100 Gewichtsteile Kaliumcarbonat in derselben Weise eingebracht und verteilt. Abschliessend wird das Granulat getrocknet und durch ein Sieb mit Maschenweite 2 mm ausgetragen.

Der Fortschritt der Reaktion des Granulats mit der Zitronensäure wird durch den jeweiligen Druckabfall festgestellt, der übrigens ein Mass für die Entwicklung von C0₂ ist. Magnesiumcarbonat reagiert träger als das Calciumcarbonat des Beispiels 1; es ist ausserdem weicher und ergibt nicht das unangenehme Gefühl einer kreidigen Konsistenz im Mund. Ausserdem bindet es bis zu 7 oder sogar 10 Moleküle Wasser pro Molekül und benötigt daher von Anfang an eine grössere Wassermenge als das Calciumcarbonat.

3026 Gewichtsteile des so hergestellten Basisgranulats werden mit folgenden Zutaten vermischt:
300 Gewichtsteile Fruchtzucker
150 Gewichtsteile Guar-Gum
400 Gewichtsteile Mannit sowie Süss- und Aromastoffe
und zu Tabletten von je 4 g verpresst.

Solche Tabletten werden als besonders angenehme Magnesiumtherapie empfunden.

### Beispiel 4:

Mit den Granulaten gemäss Beispiel 1 und 3 kann man auch eine Multivitamin-Mineral-Kautablette herstellen, indem man
500 mg des Grundgranulates (entsprechend 100 mg Calcium) wie in Beispiel 1 angeführt,
290 mg des Grundgranulates (entsprechend 35 mg Magnesium), wie im Beispiel 3 angeführt, mit
160 mg Polyvinylpolypyrrolidon
500 mg Glucose
300 mg Sorbitol und
20 mg Guar-Gum
mischt, sowie eine Vitaminmischung bestehend aus Vitamin-A-Palmitat, Thiaminmononitrat, Riboflavinphosphat-Natrium, Pyridoxinhydrochlorid, Cyanocobalamin, Calciumpantothenat, Nicotinamid, Folsäure, Biotin, Vitamin D3 sowie Vitamin C und Tocopherolacetat, sowie Süss-Stoffe und Aromen zumischt. Die Vitamindosierung kann entsprechend 10 bis 50% des RDA-Wertes betragen. Die Tablette wird dann mit einem Gewicht von 1,9 bis 2 g verpresst.

### Beispiel 5:

Das in Beispiel 3 beschriebene Magnesium-Basisgranulat ist auch besonders gut geeignet, um eine Aspirin-Kautablette zu konzipieren.

Zu einer solchen Menge des Basisgranulats, die 7,5 mmol Magnesium entspricht, kann besonders zweckmässig das folgende Mengenverhältnis zugemischt werden:
300 Gewichtsteile Aspirin
900 Gewichtsteile Xylit
300 Gewichtsteile Mannit
100 Gewichtsteile Hydrokolloide
50 Gewichtsteile Natriumcarbonat
sowie die gewünschte Menge an Süss- und Aromenstoffen und auch die entsprechende Menge von Gleit- und Press-Hilfsmitteln.

Aspirin-Kautabletten können durch die Zugabe von Natriumcarbonat auf den gewünschten pH-Bereich eingestellt werden; sie sind dann besonders angenehm einzunehmen, wobei gleichzeitig das Aspirin durch die Erdalkalisalze und das Natriumcarbonat während des Lutschens oder Kauens abgepuffert wird.

## Patentansprüche (Patentansprüche für folgende(n) Vertragsstaat(en): AT, BE, CH, LI, DE, DK, FR, GB, IT, LU, MC, NL, PT, SE)

1. Lutsch- oder Kautablette, dadurch gekennzeichnet, dass sie als Basis wenigstens 30, vorzugsweise 50 bis 70 Gewichtsprozent eines Alkali- und/oder Erdalkalisalzes wenigstens einer essbaren, organischen Säure; wenigstens 10, vorzugsweise 20 bis 40 Gewichtsprozent einer Mischung aus wenigstens einer unreagierten, essbaren organischen Säure mit wenigstens einem unreagierten Alkali- und/oder Erdalkalicarbonat und/oder -bicarbonat, sowie gegebenenfalls einen pharmazeutischen Wirkstoff und/oder andere übliche Tablettenhilfsstoffe, wie Aroma- und Süss-Stoffe, Zerfallshilfsmittel, Hydrokolloide, enthält.

2. Tablette nach Anspruch 1, dadurch gekennzeichnet, dass sie im wesentlichen das zweibasische Alkali- und/oder Erdalkalisalz einer dreibasischen, essbaren, organischen Säure, insbesondere der Zitronensäure, sowie vorzugsweise eine - insbesondere nur teilweise zum Alkali- und/oder Erdalkalisalz reagierte - essbare, organische Säure, insbesondere Äpfelsäure, enthält, die stärker als die dreibasische Säure ist.

3. Tablette nach Anspruch 1 oder 2, dadurch gekennzeichnet, dass sie - auf das Gesamttablettengewicht bezogen - 1 bis 5, vorzugsweise 2 bis 4 Gewichtsprozent wenigstens eines Hydrokolloids, insbesondere Guar-Gum enthält.

4. Tablette nach einem der vorhergehenden Ansprüche, dadurch gekennzeichnet, dass sie - auf das Gesamttablettengewicht bezogen - 1 bis 10, vorzugsweise 3 bis 7 Gewichtsprozent wenigstens eines Sprengmittels, insbesondere je etwa 3 Gewichtsprozent Stärke und quervernetztes Polyvinylpolypyrrolidon enthält.

5. Tablette nach einem der vorhergehenden Ansprüche, gekennzeichnet durch einen Gehalt von - auf das Gesamttablettengewicht bezogen - 20 bis 40, vorzugsweise 30 bis 35 Gewichtsprozent Calcium und/oder Magnesiumcarbonat; 20 bis 40, vorzugsweise 30 bis 35 Gewichtsprozent wenigstens einer essbaren, organischen Säure; und 20 bis 60, vorzugsweise 30 bis 50 Gewichtsprozent üblicher Tablettenhilfsstoffe.

6. Tablette nach einem der vorhergehenden Ansprüche, gekennzeichnet durch einen Gehalt von - auf ein Millimol Magnesium bezogen - 10 bis 80, vorzugsweise 30 bis 50 mg Acetylsalicylsäure.

7. Verfahren zur Herstellung einer Tablette nach einem der vorhergehenden Ansprüche, dadurch gekennzeichnet, dass in einem Mischkessel wenigstens ein pulverisiertes Erdalkalicarbonat oder Alkalibicarbonat mit Wasser benetzt und anschliessend unter Zufügen wenigstens einer essbaren, organischen Säure zu wenigstens 30, vorzugsweise 50 bis 70 Prozent der stöchiometrischen Menge durchreagiert wird, wobei bei einem definierten Vakuum die Reaktion bei gleichzeitiger Entfernung des entstehenden C0₂ bewirkt wird, worauf die Mischung getrocknet, vorzugsweise durch ein Sieb ausgetragen, und vor dem Verpressen zu Tabletten mit üblichen Tablettenhilfsstoffen sowie gegebenenfalls pharmazeutischen Wirkstoffen vermischt wird.

8. Verfahren nach Anspruch 7, dadurch gekennzeichnet, dass die essbare, organische Säure in mehreren, vorzugsweise zwei bis vier Einzelschritten eingebracht und zur Reaktion gebracht wird.

9. Verfahren nach Anspruch 7 oder 8, dadurch gekennzeichnet, dass nach abgeschlossener Reaktion der Mischung noch wenigstens ein Alkalicarbonat oder -bicarbonat und/oder wenigstens ein Hydrokolloid, insbesondere Guar-Gum, zugesetzt und unter schwingendem Mischen verteilt werden.

## Patentansprüche (Patentansprüche für folgende(n) Vertragsstaat(en): GR, ES)

1. Verfahren zur Herstellung einer Lutsch- oder Kautablette, dadurch gekennzeichnet, dass in einem Mischkessel wenigstens ein pulverisiertes Alkalibicarbonat oder Erdalkalicarbonat mit Wasser benetzt und anschliessend unter Zufügen wenigstens einer essbaren, organischen Säure zu wenigstens 30, vorzugsweise 50 bis 70 Prozent der stöchiometrischen Menge durchreagiert wird, wobei bei einem definierten Vakuum die Reaktion bei gleichzeitiger Entfernung des entstehenden CO₂ bewirkt wird, worauf die Mischung getrocknet, vorzugsweise durch ein Sieb ausgetragen, und vor dem Verpressen zu Tabletten gegebenenfalls mit pharmazeutischen wirkstoffen und/oder üblichen Tablettenhilfsstoffen, wie Aroma- und Süss-Stoffen, Zerfallshilfsmitteln, Hydrokolloiden, vermischt wird.

2. Verfahren nach Anspruch 1, dadurch gekennzeichnet, dass die essbare, organische Säure in mehreren, vorzugsweise zwei bis vier Einzelschritten eingebracht und zur Reaktion gebracht wird.

3. Verfahren nach Anspruch 1 oder 2, dadurch gekennzeichnet, dass nach abgeschlossener Reaktion der Mischung noch wenigstens ein Alkalicarbonat oder -bicarbonat und/oder wenigstens ein Hydrokolloid, insbesondere Guar-Gum, zugesetzt und unter schwingendem Mischen verteilt werden.

4. Verfahren nach einem der vorhergehenden Ansprüche, dadurch gekennzeichnet, dass die Säure dreibasisch, insbesondere Zitronensäure, ist und - zumindest teilweise - zu ihrem zweibasischem Alkali- und/oder Erdalkalisalz reagiert wird.

5. Verfahren nach einem der vorhergehenden Ansprüche, dadurch gekennzeichnet, dass eine weitere - vorzugsweise nur teilweise zum Alkali- und/oder Erdalkalisalz reagierte - essbare, organische Säure, insbesondere Äpfelsäure, zugesetzt wird, die stärker als die dreibasische Säure ist.

6. Verfahren nach einem der vorhergehenden Ansprüche, dadurch gekennzeichnet, dass die Mischung - auf das Gesamttablettengewicht bezogen - 1 bis 5, vorzugsweise 2 bis 4 Gewichtsprozent wenigstens eines Hydrokolloids, insbesondere Guar-Gum enthält.

7. Verfahren nach einem der vorhergehenden Ansprüche, dadurch gekennzeichnet, dass die Mischung - auf das Gesamttablettengewicht bezogen - 1 bis 10, vorzugsweise 3 bis 7 Gewichtsprozent wenigstens eines Sprengmittels, insbesondere je etwa 3 Gewichtsprozent Stärke und quervernetztes Polyvinylpolypyrrolidon, enthält.

8. Verfahren nach einem der vorhergehenden Ansprüche, dadurch gekennzeichnet, dass die Mischung - auf das Gesamttablettengewicht bezogen - 20 bis 40, vorzugsweise 30 bis 35 Gewichtsprozent Calcium- und/oder Magnesiumcarbonat; 20 bis 40, vorzugsweise 30 bis 35 Gewichtsprozent wenigstens einer essbaren, organischen Säure; und 20 bis 60, vorzugsweise 30 bis 50 Gewichtsprozent üblicher Tablettenhilfsstoffe, enthält.

9. Verfahren nach einem der vorhergehenden Ansprüche, mit einem Gehalt an Magnesiumcarbonat, dadurch gekennzeichnet, dass die Michung - auf ein Millimol Magnesium bezogen - 10 bis 80, vorzugsweise 30 bis 50 mg Acetylsalicylsäure enthält.

## Claims (Claims for the following Contracting State(s): AT, BE, CH, LI, DE, DK, FR, GB, IT, LU, MC, NL, PT, SE)

1. Lozenge or chewable tablet, characterised in that it contains, as the base, at least 30, preferably 50 to 70, percent by weight of an alkali metal and/or alkaline earth metal salt of at least one edible, organic acid; at least 10, preferably 20 to 40, percent by weight of a mixture of at least one unreacted, edible organic acid with at least one unreacted alkali metal or alkaline earth metal carbonate and/or bicarbonate and, if required, a pharmaceutical active substance and/or other conventional tablet excipients, such as flavours and sweeteners, disintegrants, hydrocolloids.

2. Tablet according to Claim 1, characterised in that it essentially contains the dibasic alkali metal and/or alkaline earth metal salt of a tribasic, edible, organic acid, in particular of citric acid, and preferably an edible, organic acid, which has in particular only partially reacted to give the alkali metal and/or alkaline earth metal salt, in particular malic acid, which is stronger than the tribasic acid.

3. Tablet according to Claim 1 or 2, characterised in that it contains - based on the total tablet weight - 1 to 5, preferably 2 to 4, percent by weight of at least one hydrocolloid, in particular guar gum.

4. Tablet according to any of the preceding Claims, characterised in that it contains - based on the total tablet weight - 1 to 10, preferably 3 to 7, percent by weight of at least one disintegrant, in particular about 3 percent by weight each of starch and of crosslinked polyvinylpyrrolidone.

5. Tablet according to any of the preceding Claims, characterised by a content of - based on the total tablet weight - 20 to 40, preferably 30 to 35, percent by weight of calcium carbonate and/or magnesium carbonate, 20 to 40, preferably 30 to 35, percent by weight of at least one edible, organic acid and 20 to 60, preferably 30 to 50, percent by weight of conventional tablet excipients.

6. Tablet according to any of the preceding Claims, characterised by a content of - based on one millimole of magnesium - 10 to 80, preferably 30 to 50, mg of acetylsalicylic acid.

7. Process for the preparation of a tablet according to any of the preceding Claims, characterised in that, in a mixing vessel, at least one powdered alkaline earth metal carbonate or alkali metal bicarbonate is wet with water and then, with the addition of at least one edible, organic acid, is reacted to an extent of at least 30, preferably 50 to 70, percent of the stoichiometric amount, the reaction being carried out at a defined vacuum with simultaneous removal of the CO₂ formed, after which the mixture is dried, discharged, preferably through a sieve, mixed with conventional tablet excipients and, if required, pharmaceutical active substances and then compressed to give tablets.

8. Process according to Claim 7, characterised in that the edible, organic acid is introduced in several individual steps, preferably two to four individual steps, and is reacted.

9. Process according to Claim 7 or 8, characterised in that, after the reaction of the mixture is complete, at least one alkali metal carbonate or bicarbonate and/or at least one hydrocolloid, in particular guar gum, are added and the are distributed with vibratory mixing.

## Claims (Claims for the following Contracting State(s): GR, ES)

1. Process for the preparation of a lozenge or chewable tablet, characterized in that, in a mixing vessel, at least one powdered alkali metal bicarbonate or alkaline earth metal carbonate is wet with water and then, with the addition of at least one edible, organic acid, reacted to an extent of at least 30, preferably 50 to 70, percent of the stoichiometric amount, the reaction being effected by simultaneous removal of the resulting CO₂ at a defined vacuum, after which the mixture is dried, preferably discharged through a sieve and, before being compressed to tablets, optionally mixed with pharmaceutical active substances and/or conventional tablet excipients, such as flavours and sweeteners, disintegrants and hydrocolloids.

2. Process according to Claim 1, characterized in that the edible organic acid is introduced in several, preferably two to four, individual steps and is reacted.

3. Process according to Claim 1 or 2, characterized in that, after the reaction of the mixture has ended, at least one alkali metal carbonate or bicarbonate and/or at least one hydrocolloid, in particular guar gum, are added and are distributed with vibratory mixing.

4. Process according to any of the preceding Claims, characterized in that the acid is tribasic, in particular is citric acid, and is reacted - at least partially - to give its dibasic alkali metal salt and/or alkaline earth metal salt.

5. Process according to any of the preceding Claims, characterized in that a further edible, organic acid - preferably reacted only partially to give the alkali metal salt and/or alkaline earth metal salt - in particular malic acid, which is stronger than the tribasic acid is added.

6. Process according to any of the preceding Claims, characterized in that the mixture contains - based on the total tablet weight - 1 to 5, preferably 2 to 4, percent by weight of at least one hydrocolloid, in particular guar gum.

7. Process according to any of the preceding Claims, characterized in that the mixture contains - based on the total tablet weight - 1 to 10, preferably 3 to 7, percent by weight of at least one disintegrant, in particular about 3 percent by weight each of starch and crosslinked polyvinylpyrrolidone.

8. Process according to any of the preceding Claims, characterized in that the mixture contains - based on the total tablet weight - 20 to 40, preferably 30 to 35, percent by weight of calcium carbonate and/or magnesium carbonate; 20 to 40, preferably 30 to 35, percent by weight of at least one edible organic acid; and 20 to 60, preferably 30 to 50, percent by weight of conventional tablet excipients.

9. Process according to any of the preceding Claims, having a content of magnesium carbonate, characterized in that the mixture contains - based on one millimole of magnesium - 10 to 80, preferably 30 to 50, mg of acetylsalicylic acid.

## Revendications (Revendications pour l'(les) Etat(s) contractant(s) suivant(s): AT, BE, CH, LI, DE, DK, FR, GB, IT, LU, MC, NL, PT, SE)

1. Comprimé à sucer ou à croquer, caractérisé en ce qu'il contient à la base, pour l'essentiel, au moins 30, de préférence 50 à 70 % en poids d'un sel alcalin et/ou alcalino-terreux d'au moins un acide organique comestible ; au moins 10, de préférence 20 à 40 % en poids d'un mélange d'au moins un acide organique comestible qui n'a pas réagit et d'un carbonate et/ou d'un bicarbonate alcalin et/ou alcalino-terreux qui n'a pas réagit, ainsi que, le cas échéant, d'une substance pharmaceutique et/ou d'autres additifs de comprimés habituels, tels que des aromates et du sucre, des désintégrants, des hydrocolloïdes.

2. Comprimé selon la revendication 1, caractérisé en ce qu'il contient pour l'essentiel le sel alcalin et/ou alcalino-terreux dibasique d'un triacide organique comestible, notamment de l'acide citrique, ainsi que, de préférence, un acide organique comestible qui n'a notamment que partiellement réagit au sel alcalin et/ou alcalino-terreux, en particulier de l'acide malique qui est est plus fort que l'acide tribasique.

3. Comprimé selon la revendication 1 ou 2, caractérisé en ce qu'il contient en référence au poids total du comprimé 1 à 5, de préférence 2 à 4 % en poids d'au moins un hydrocolloïde, notamment de l'agar-agar.

4. Comprimé selon l'une des revendications précédentes, caractérisé en ce qu'il contient en référence au poids total du comprimé 1 à 10, de préférence 3 à 7 % en poids d'un désintégrant, notamment environ 3 % en poids d'amidon et de polyvinylpolypyrrolidone à réticulation transversale, respectivement.

5. Comprimé selon l'une des revendications précédentes, caractérisé par une teneur en calcium et/ou en carbonate de magnésium en référence au poids total du comprimé de 20 à 40, de préférence 30 à 35 % en poids ; par une teneur en au moins un acide organique comestible de 20 à 40, de préférence de 30 à 35 % en poids ; et par une teneur en additifs habituels pour comprimés de 20 à 60, de préférence de 30 à 50 % en poids.

6. Comprimé selon l'une des revendications précédentes caractérisé par une teneur en acide acétylsalicylique en référence à une millimole de magnésium de 10 à 80, de préférence de 30 à 50 mg.

7. Procédé pour produire un comprimé selon l'une des revendications précédentes, caractérisé en ce que, dans une cuve de mélange, au moins un carbonate alcalino-terreux ou un bicarbonate alcalin réduit en poudre est humecté avec de l'eau puis réagit à concurrence d'au moins 30, de préférence 50 à 70 % de la quantité stoechiométrique après y avoir ajouté au moins un acide organique comestible, sachant que, pour un vide défini, la réaction se fait tout en éliminant le CO₂, produit, en ce que le mélange est ensuite séché, de préférence tamisé et, avant de former les comprimés, mélangé avec des additifs habituels pour comprimés ainsi qu'avec des substances pharmaceutiques actives, le cas échéant.

8. Procédé selon la revendication 7, caractérisé en ce que l'acide organique comestible est incorporé en plusieurs étapes, de préférence deux à quatre, puis est amené à réagir.

9. Procédé selon la revendication 7 ou 8, caractérisé en ce que, une fois la réaction du mélange terminée, on incopore encore au moins un carbonate ou un bicarbonate alcalin et/ou au moins un hydrocolloïde, notamment de l'agar-agar, et en ce qu'on les répartit par mélange pendulaire.

## Revendications (Revendications pour l'(les) Etat(s) contractant(s) suivant(s): GR, ES)

1. Procédé de préparation d'un comprimé à sucer ou à mâcher, caractérisé en ce que dans un récipient de mélange, on humecte avec de l'eau au moins un bicarbonate alcalin ou un carbonate alcalino-terreux pulvérisé, puis on le fait réagir complètement en ajoutant au moins un acide organique comestible à raison de 30, de préférence de 50 à 70 % de la quantité stoechiométrique, la réaction étant effectuée sous un vide déterminé avec élimination simultanée du CO₂ formé, après quoi on sèche le mélange, on l'évacue de préférence à travers un tamis, et avant le pressage en comprimés, on le mélange le cas échéant avec des substances actives pharmaceutiques et/ou des adjuvants habituels pour comprimés tels que des substances aromatiques et des édulcorants, des désintégrants, des hydrocolloïdes.

2. Procédé selon la revendication 1, caractérisé en ce que l'acide organique comestible est introduit et mis à régir en plusieurs, de préférence en deux à quatre stades séparés.

3. Procédé selon les revendications 1 ou 2, caractérisé en ce que lorsque la réaction est terminée, on ajoute encore au mélange au moins un carbonate ou bicarbonate alcalin et/ou au moins un hydrocolloïde, en particulier de la gomme guar, et on les disperse en les mélangeant dans un mélangeur vibrant.

4. Procédé selon une des revendications précédentes, caractérisé en ce que l'acide est un triacide, en particulier l'acide citrique, et est mis à réagir au moins partiellement pour donner son sel alcalin et/ou alcalino-terreux dibasique.

5. Procédé selon une des revendications précédentes, caractérisé en ce qu'on ajoute un autre acide organique comestible, n'ayant de préférence réagi que partiellement pour donner le sel alcalin et/ou alcalino-terreux, en particulier l'acide malique, qui est plus fort que le triacide.

6. Procédé selon une des revendications précédentes, caractérisé en ce que le mélange contient, par rapport au poids total du comprimé, 1 à 5, de préférence 2 à 4 % en poids d'au moins un hydrocolloïde, en particulier de la gomme guar.

7. Procédé selon une des revendications précédentes, caractérisé en ce que le mélange contient, par rapport au poids total du comprimé, de 1 à 10, de préférence de 3 à 7 % en poids d'au moins un désintégrant, en particulier environ 3 % en poids d'amidon et 3 % en poids de polyvinylpyrrolidone réticulée transversalement.

8. Procédé selon une des revendications précédentes, caractérisé en ce que le mélange contient, par rapport au poids total du comprimé, 20 à 40, de préférence 30 à 35 % en poids de carbonate de calcium et/ou de magnésium, 20 à 40, de préférence 30 à 35 % en poids d'au moins un acide organique comestible, et 20 à 60, de préférence 30 à 50 % en poids de substances auxiliaires ordinaires pour comprimés.

9. Procédé selon une des revendications précédentes, contenant du carbonate de magnésium, caractérisé en ce que le mélange contient, pour une millimole de magnésium, 10 à 80, de préférence 30 à 50 mg d'acide acétylsalicylique.
